# EUROPÄISCHE PATENTSCHRIFT

(11) **EP 1 586 563 B1**
(45) Veröffentlichungstag und Bekanntmachung des Hinweises auf die Patenterteilung: **09.12.2015**
(21) Anmeldenummer: 05007334.5
(22) Anmeldetag: 05.04.2005
(51) Int. Cl.: C07C 381/02

(54) **Herstellung von Thioschwefelsäurederivaten**
Preparation of thiosulphuric acid derivatives
Préparation de dérivés d'acide thiosulfurique

(30) Priorität: 15.04.2004 DE 102004018193
(43) Veröffentlichungstag der Anmeldung: 19.10.2005
(73) Patentinhaber: LANXESS Deutschland GmbH, 50569 Köln (DE)
(72) Erfinder: Buding, Hartmuth, Dr., 52445 Titz (DE)

(56) Entgegenhaltungen:
- EP-A- 0 601 303
- DATABASE CAPLUS CHEMICAL ABSTRACTS SERVICE, COLUMBUS, OHIO, US; XP002391744 Database accession no. 1950:17518 & YA.G. MAZOVER: "New derivatives of disulphones. I. Homologues and analogues of sulphonal" ZHURNAL OBSHCHEI KHIMII, Bd. 19, 1949, Seiten 843-848, NAUKA, MOSCOW, RU

## Beschreibung

Die Erfindung betrifft ein Verfahren zur Herstellung von Salzen von S-Alkylestern der Thioschwefelsäure.

Die Herstellung von Salzen von S-Alkylestern der Thioschwefelsäure (Bunte-Salze) ist prinzipiell bekannt (vgl. z.B. B. Milligan und J.M. Swan in Rev. Pure and Applied Chemistry 12, 1962, Seiten 72 bis 94, und H. Distler in Angew. Chem. 79, 1967, Seiten 520 bis 529).

Nach B. Milligan et al. werden Bunte-Salze im Allgemeinen durch Umsetzung von Alkylhalogeniden mit Natriumthiosulfat in einer siedenden Lösung von 50%igem wässrigen Ethanol hergestellt (Seite 74, rechte Kolonne, 3. Absatz). Nur im Falle von wasserlöslichen Alkylhalogeniden wird Wasser als alleiniges Lösungsmittel verwendet.

Die EP-A 70 143 beschreibt als Lösungsmittel zur Herstellung von kristallwasserhaltigem 1,6-Hexamethylen-bis(Natriumthiosulfat) eine 1:1-Mischung aus Wasser und Ethanol. Als erfinderische Lösungsmittel werden jedoch Ethylen- und Diethylenglykol offenbart (Seite 16, Zeilen 2 bis 9). Eventuell vorhandenes Wasser im Ethylen- oder Diethylenglykol, als auch Kristallwasser im Thiosulfat, wird vor Zugabe des Dihalogenids durch Destillation entfernt. Die in den glykolischen Lösungsmitteln gelösten Reaktionsprodukte werden durch Hinzufügen von bestimmten Lösungsmitteln ausgefällt. Als ein solches Lösungsmittel (Fällungsmittel) wird Isopropanol genannt. Nachteil dieses Verfahrens ist, dass zum einen die Glykole als auch das Thiosulfat vor Beginn der Reaktion durch Destillation entwässert werden müssen und dass zum anderen große Mengen an organischen Lösungsmitteln (Glykole und Fällungsmittel) gehandhabt werden müssen. Am Ende der Synthese sind die Glykole und das Fällungsmittel destillativ aufzuarbeiten, was aufwändig ist.

In der DE-A 22 56 511 wird u.a. auf Seite 39 die Herstellung eines Bis-Bunte-Salzes wie folgt beschrieben: Man setzt 1,4-Dichlorbuten-2 in wässrigem Ethanol mit Natriumthiosulfatpentahydrat bei Siedetemperatur des Lösungsmittelgemisches um und entfernt dann nach beendeter Reaktion das Ethanol durch Destillation. Das in Wasser gelöste Bis-Bunte-Salz wird dann ohne Isolierung als Edukt für eine weitere Synthesestufe verwendet. Nachteil dieses Verfahrens ist die notwendige destillative Abtrennung des Ethanols aus dem Reaktionsgemisch.

Eine Nacharbeitung der Lehre der DE-A 22 56 511 am Beispiel von 1,6-Dichlorhexan hat folgendes ergeben: Beim Abdestillieren von Ethanol aus dem Reaktionsgemisch der Umsetzung von 1,6-Dichlorhexan mit Natriumthiosulfatpentahydrat in einem Gemisch aus Ethanol und Wasser wurde beobachtet, dass das Ethanol übelriechende Nebenprodukte aus dem Reaktionsgemisch mitschleppt und so das Destillat ohne zusätzliche, aufwendige Reinigung desselben unbrauchbar macht, und damit einer Verbrennung zugeführt werden muss. Darüber hinaus wurde die Destillationskolonne beim Abdestillieren des Ethanols in einigen Fällen von den übelriechenden Nebenprodukten durch schmierige Ablagerungen verschmutzt, was eine Kolonnenreinigung erforderlich machte.

In den Patentanmeldungen EP-A 385 072 und EP-A 432 417 ist die Synthese von Bis-Bunte-Salzen in sehr allgemeiner Weise am Beispiel der Umsetzung von 1,2-Dichlorethan mit Natriumthiosulfat in wässriger Lösung angeführt. Über vorteilhaft anzuwendende Reaktionsparameter werden keine Angaben gemacht. Auch werden keine Ausführungsbeispiele für die Synthese gegeben. Eigene Versuche zur Nacharbeitung des allgemeinen Hinweises der EP-A 432 417 am Beispiel von 1,6-Dichlorhexan ergaben einen Umsatz des eingesetzten Dihalogenids von nur ca. 89 % (vgl. Beispiel 6). Derart niedrige Umsätze des Dihalogenids machen eine destillative Abtrennung desselben aus dem Reaktionsgemisch notwendig, gefolgt von einer Aufarbeitung zwecks Rückführung. Zum anderen ist zu vermuten, dass bei derart niedrigen Umsätzen des α,ω-Dihalogenalkans, trotz Überschuss an Natriumthiosulfat, neben dem gewünschten Bis-Bunte-Salz in erheblichem Umfang auch das nicht gewünschte Produkt entsteht, bei dem nur ein Halogenatom durch die S-SO₃Na-Gruppe ersetzt wurde.

EP-A 0 601 303 offenbart ein Verfahren zur Herstellung des Bisnatriumsalzes der 1,6-Hexanbisthiosulfonsäure durch Umsetzung von 1,6-Dichlorhexan mit Natriumthiosulfatpentahydrat in Wasser bei Rückfluss und einem pH-Wert von 7 bis 8. Eine Einstellung des pH-Werts während der Umsetzungs ist nicht offenbart.

Aufgabe der vorliegenden Erfindung ist es, ein einfaches, technisch gut praktizierbares, umwelt- und resourcenschonendes Verfahren zur Herstellung von Salzen von S-Alkylestern der Thioschwefelsäure in Wasser, ohne Zusatz von Alkoholen und/oder Glykolen, mit hohen Umsätzen der zugrunde liegenden α,ω-Dihalogenalkane und mit technisch guten Ausbeuten und hohen Gehalten an den Salzen der S-Alkylestern der Thioschwefelsäure bereitzustellen.

Mit den bekannten Herstellverfahren kann das gesetzte Ziel nicht erreicht werden.

Es wurde nun gefunden, dass sich in Wasser praktisch unlösliche α,ω-Dihalogenalkane in Wasser als Reaktionsmedium in Abwesenheit von Alkoholen und/oder Glykolen mit Thiosulfationen unter Einhaltung eines bestimmten pH-Wert-Bereichs in hohen Umsätzen zu Bis-Bunte-Salzen mit technisch guten Ausbeuten und hohen Gehalten umsetzen lassen.

Gegenstand der vorliegenden Erfindung ist daher ein Verfahren zur Herstellung der Verbindungen der Formel (I)

Me¹ O₃S S-(CH₂)ₙ-S SO₃ Me² (I),

worin Me¹ und Me² gleich oder verschieden sind und einwertige Metallionen oder Ammoniumionen bedeuten und n eine ganze Zahl von 2 bis 8 darstellt,
dadurch gekennzeichnet, dass man Verbindungen der Formel (II)

X-(CH₂)ₙ-X (II),

worin X Halogen bedeutet und n die gleiche Bedeutung wie in der Formel (I) hat,
mit Thiosulfationen bei einer Reaktionstemperatur von 80°C bis 150°C umsetzt, wobei die Umsetzung in Wasser ohne Zusatz von Alkoholen und/oder Glykolen in einem pH-Wert-Bereich von 3 bis 9,8 durchgeführt wird.

Die Laufzahl n in der Formel (I) stellt eine ganze Zahl von 2 bis 8, bevorzugt von 3 bis 8, ganz besonders bevorzugt von 4 bis 8 dar.

In der Formel (I) sind Me¹ und Me² gleich oder verschieden und bedeuten einwertige Metallionen oder Ammoniumionen. Bevorzugt sind Me¹ und Me² gleich und bedeuten Alkalimetall- oder Ammoniumionen, bevorzugt Alkalimetallionen. Von den Alkalimetallionen sind Natrium- und Kaliumionen bevorzugt, ganz besonders bevorzugt sind Natriumionen.

In der Formel (II) steht X vorzugsweise für Chlor oder Brom. Die erfindungsgemäßen Verbindungen der Formel (II) können auch gemischte Halogenide darstellen, d.h. sie können gleichzeitig verschiedene Halogenatome enthalten.

Die Umsetzung der Verbindungen der Formel (II) wird in Wasser ohne Zusatz von Alkoholen und/oder Glykolen mit Thiosulfationen in einem geschlossenen oder offenen System durchgeführt. Die Thiosulfationen werden bevorzugt in Form von Alkalithiosulfaten oder in Form von Ammoniumthiosulfat eingesetzt. Aus der Reihe der Alkalithiosulfaten ist aus Gründen der Verfügbarkeit die Verwendung von Natriumthiosulfat bzw. Natriumthiosulfatpentahydrat bevorzugt, es können prinzipiell aber auch Lithium-, Kalium-, Cäsium- oder Rubidiumthiosulfat, oder Gemische hieraus, verwendet werden.

Sofern die erfindungsgemäße Reaktionsmischung unmittelbar vor Beginn der Umsetzung bei Raumtemperatur nicht einen pH-Wert im Bereich von 3 bis 9,8 aufweist, ist es vorteilhaft, einen pH-Wert in diesem Bereich durch Zugabe von Säuren oder Basen entsprechend einzustellen, vorzugsweise mit Mineralsäuren, wie z.B. Salzsäure, oder durch Laugen von Alkalihydroxiden, wie z.B. Natriumhydroxid, um dann im Anschluss die erfindungsgemäße Reaktion zu starten.

Der pH-Wert der Reaktionsmischung wird während der Reaktion im Sinne der Erfindung im Bereich von 3 bis 9,8, bevorzugt von 4 bis 9,4, ganz besonders bevorzugt von 5 bis 9 gehalten bzw. eingestellt.

Es wurde festgestellt, dass bei der Umsetzung der Verbindungen der Formel (II) mit Thiosulfationen die Reaktionsmischung mit fortscheitender Reaktion immer saurer wird.

Die Einstellung des pH-Wertes während der erfindungsgemäßen Umsetzung der Verbindungen der Formel (II) erfolgt dann durch Zugabe von organischen oder anorganischen Basen oder Mischungen hieraus. Als organische Basen für die Erfindung kommen bevorzugt tertiäre Alkylamine in Frage mit einem Kohlenwasserstoffrest von 2 bis 14 Kohlenstoffatomen. Als anorganische Basen für die Erfindung kommen bevorzugt Alkali- oder Erdalkalihydroxide, ganz besonders bevorzugt Alkalihydroxide, in Frage. Aus der Reihe der Alkalihydroxide ist Natrium- oder Kaliumhydroxid bevorzugt, ganz besonders bevorzugt ist Natriumhydroxid. Von den Basen zur Einstellung des erfindungsgemäßen pH-Wert-Bereichs sind die anorganischen Basen bevorzugt. Die erfindungsgemäßen pH-Werte können aber auch durch ein Puffersystem, das keinen negativen Einfluss auf die erfindungsgemäße Umsetzung hat und gut abwasserverträglich ist, wie z.B. Natriumhydrogencarbonat, eingestellt werden. Die optimale Dosierung kann leicht durch Versuche herausgefunden werden.

Zur Einstellung des pH-Wertes bei der erfindungsgemäßen Umsetzung der Verbindungen der Formel (II) mit Thiosulfationen kann die Base als solche oder auch als wässrige Lösung zugesetzt werden. Bevorzugt wird die Base als möglichst verdünnte, wässrige Lösung zum Reaktionsgemisch zugegeben, um eine schnelle Verteilung und damit eine schnelle pH-Wert-Einstellung zu erlangen. Im Falle von Natrium- und Kaliumhydroxid ist eine 0,01 bis 15 gew.-%ige, bevorzugt 0,1 bis 10 gew.-%ige, wässrige Lösung besonders geeignet. Für andere Basen kann die optimale Konzentration leicht durch Versuche herausgefunden werden.

Die erfindungsgemäße Menge an einzusetzendem Thiosulfat beträgt ca. 200 bis 250 Mol-%, bevorzugt 200 bis 240 Mol-%, ganz besonders bevorzugt 200 bis 230 Mol-%, bezogen auf die Mole an eingesetzten Verbindungen der Formel (II). Kleinere Thiosulfatmengen können zwar eingesetzt werden, sie gehen jedoch zu Lasten der Ausbeute und der Gehalte an Bis-Bunte-Salz. Größere Thiosulfatmengen können ebenfalls eingesetzt werden, sie sind jedoch nicht nur aus ökologischer Sicht im Hinblick auf das Abwasser unerwünscht, sondern auch im Hinblick auf sich bildende Nebenprodukte.

Die Menge an einzusetzendem Wasser als Reaktionsmedium bei der erfindungsgemäßen Umsetzung der Verbindungen der Formel (II) mit Thiosulfat ist nicht kritisch. Vor dem Hintergrund möglichst kleiner Abwassermengen bei der Isolierung der Verbindungen der Formel (I) oder in einer sich anschließenden Reaktionsstufe ohne Isolierung der Verbindungen der Formel (I) sollte die Wassermenge bei der Umsetzung der Verbindungen der Formel (II) möglichst klein gewählt werden. Die Wassermenge ist vorteilhaft jedoch so groß zu wählen, dass das eingesetzte Thiosulfat, als auch die sich bildenden Verbindungen der Formel (I), in der Reaktionsmischung bei Reaktionstemperatur gerade noch gelöst sind.

Die erfindungsgemäßen Reaktionstemperaturen für das erfindungsgemäße Herstellverfahren liegen bei ca. 80° bis 150°C, bevorzugt bei 85° bis 140°C, ganz besonders bevorzugt bei 90° bis 130°C. Im offenen, drucklosen System wird vorteilhaft bei Siedetemperatur des Reaktionsgemisches gearbeitet. Der Umsatz für die Verbindungen der Formel (II) bei der Reaktion mit Thiosulfationen im Sinne der Erfindung beträgt mindestens 94 %, bevorzugt mindestens 96 %, ganz besonders bevorzugt mindestens 98 %. Die Umsatzbestimmung für die Verbindungen der Formel (II) kann z.B. mittels Gaschromatographie (interner oder externer Standard) am Reaktionsgemisch erfolgen. Die Reinausbeute (= Ausbeute x Gehalt) an den Salzen der S-Alkylester der Thioschwefelsäure beträgt nach dem erfindungsgemäßen Herstellverfahren mindestens 70 %, bevorzugt mindestens 75 %, ganz besonders bevorzugt mindestens 80 %.

Da die Verbindungen der Formel (II) in Wasser praktisch unlöslich sind, ist bei deren erfindungsgemäßen Umsetzung auf eine gute Durchmischung der Reaktionsmischung, z.B. durch Rühren, zu achten.

Die erfindungsgemäßen Verbindungen der Formel (I) können z.B. in Form ihres wässrigen Reaktionsgemisches nach abgeschlossener Umsetzung der Verbindungen der Formel (II) als Bausteine für chemische Synthesen eingesetzt werden. In isolierter Form können die erfindungsgemäßen Verbindungen der Formel (I), gegebenenfalls kristallwasserhaltig, z.B. zur Vernetzung von Dienkautschuken in Kombination mit Schwefel und Beschleuniger eingesetzt werden.

Bei Bedarf können die erfindungsgemäßen Verbindungen der Formel (I) mit Metallkationen unter Austausch der erfindungsgemäßen Kationen Me¹ und Me² umgesetzt werden. Dies kann z.B. in wässriger Lösung mit Hilfe von entsprechend mit Metallkationen belegten Kationenaustauscherharzen erfolgen.

### Beispiele

### Beispiel 1

In einer mit Stickstoff gespülten 2 1-Vierhalskolbenrührapparatur mit Innenthermometer, Rückflusskühler mit Blasenzähler und pH-Elektrode wurden unter Rühren 300 g vollentsalztes Wasser und 136,5 g (0,55 mol) Natriumthiosulfatpentahydrat vorgelegt. Nachdem das Thiosulfat gelöst war, gab man 38,8 g (0,25 mol) 1,6-Dichlorhexan hinzu. Die schwach saure Mischung wurde mit einigen Tropfen 2,5%iger Natronlauge auf pH 7,2 eingestellt. Man spülte das Reaktionsgefäß nochmals kurz mit Stickstoff und kochte dann die Mischung 9 h lang am Rückfluss, wobei während dieser Zeit der pH-Wert der Reaktionsmischung durch Zugabe von 2,5%iger Natronlauge mittels einer Dosierpumpe auf 7,2 ± 0,1 gehalten wurde (pH-Elektrode). Nach jeweils 6 und 8 Stunden der Reaktion wurde der Blasenzähler vom Rückflusskühler kurz abgenommen. Sodann wurden in den Rückflusskühler von oben je einmal ca. 5 ml vollentsalztes Wasser mit einer Spritzflasche eingespritzt um eventuell nicht zurückgetropftes 1,6-Dichlorhexan in den Kolben zurückzuspülen. Nach beendeter Reaktionszeit waren ca. 15 ml an 2,5%iger Natronlauge für die pH-Wert-Steuerung verbraucht. Die Reaktionsmischung war klar und homogen. Nach kurzem Abkühlen wurde dem Reaktionsgemisch eine Probe zur Umsatzbestimmung von 1,6-Dichlorhexan durch Gaschromatographie (GC) entnommen. Die GC-Analyse mit internem Standard ergab einen Restgehalt an 1,6-Dichlorhexan von <10 ppm, was einem Umsatz an 1,6-Dichlorhexan von >99,9% entspricht.

Über Nacht kristallisierte aus dem natriumthiosulfat- und stark natriumchloridhaltigen Reaktionsgemisch etwas flockiger Niederschlag an 1,6-Hexamethylen-bis(Natriumthiosulfat)-Dihydrat aus. Dieser wurde mittels einer Glassinterfritte isoliert, 2 mal mit je ca. 25 ml Ethanol gewaschen und im Vakuumtrockenschrank bei 50°C getrocknet. Es wurden ca. 3,7 g feines Kristallpulver erhalten, das elementaranalytisch, bezogen auf 1,6-Hexamethylen-bis(Natriumthiosulfat)-Dihydrat, noch nicht ganz analysenrein war:

| | | |
|---|---|---|
| C ber.: 18,46% | H ber.: 4,13% | S ber.: 32,85% |
| C gef.: 17,3/17,3% | H gef.: 3,5/3,5% | S gef.: 32,0/32,9 % |

Das IR-Spektrum (KBr) dieses rohen Kristallpulvers stand in Einklang mit der erwarteten Struktur:

| | |
|---|---|
| 3564, 3458 cm⁻¹ | (Kristallwasser) |
| 2927, 2858 cm⁻¹ | (CH-Valenz) |
| 1619 cm⁻¹ | (OH-Deformation) |
| 1465 cm⁻¹ | (CH₂, asymetrische Deformation) |
| 1214, 1048, 654 cm⁻¹ | (S-SO₃⁻¹) |

Durch Einengen der Mutterlauge kann weiteres Produkt gewonnen werden.

Zur Ausbeutebestimmung wurde die Reaktion nochmals wiederholt. Eine GC-Analysenprobe wurde dem Reaktionsgemisch jedoch nicht entnommen. Die Reinausbeute an 1,6-Hexamethylenbis(Natriumthiosulfat)-Dihydrat war bei dieser Umsetzung ca. 93,3%¹⁾.
¹⁾ Die Reinausbeute an 1,6-Hexamethylen-bis(Natriumthiosulfat)-Dihydrat wurde indirekt aus der Ausbeute einer nachfolgenden Umsetzung der erhaltenen Reaktionslösung wie folgt bestimmt: Durch Umsetzung der erfindungsgemäßen Reaktionslösung mit einer wässrigen Natriumdibenzyldithiocarbamat-Lösung (NaBEC-Lösung) wurden nach Aufarbeitung ca. 156,9 g Kristalle (90,5% der Theorie) erhalten. Der Gehalt an 1,6-Bis(N,N-dibenzylthiocarbamoyldithio)hexan wurde mittels HPLC (externer Standard) zu ca. 96% bestimmt. Daraus ergibt sich eine Reinausbeute an 1,6-Bis(N,N-dibenzylthiocarbamoyldithio)hexan von ca. 86,9%. Durch Umsetzung von 1,6-Hexamethylen-bis(Natriumthiosulfat)-Dihydrat mit einem Gehalt von 98% (Duralink® HTS der Fa. Flexsys/Belgien) in wässriger Lösung mit NaBEC-Lösung unter gleichen Reaktionsbedingungen wie bei der zuvor geschilderten Umsetzung erhielt man 1,6-Bis(N,N-dibenzylthiocarbamoyldithio) hexan in einer Ausbeute von 96% (der Theorie) und mit einem Gehalt von ca. 99%, was einer Reinausbeute von 96% x 0,99 x 0,98 = 93,1% entspricht. Damit muss die Reinausbeute an 1,6-Hexamethylen-bis(Natriumthiosulfat)-Dihydrat bei der erfindungsgemäßen Umsetzung von 1,6-Dichlorhexan mit Natriumthiosulfatpentahydrat ca. 93,3% (86,9 x 1/0,931 = 93,3%) betragen haben.

### Beispiele 2 bis 5

Man arbeitete wie in Beispiel 1, führte jedoch die Umsetzung des 1,6-Dichlorhexans bei einem konstanten pH-Wert von 5, 6, 8 und 9 durch. Vor dem Start der jeweiligen Reaktion wurde die schwach saure Mischung mit 2,5%iger Natronlauge auf den pH-Wert von 5, 6, 8 und 9 eingestellt (Messung mit pH-Elektrode).

| | Beispiel 2 | Beispiel 3 | Beispiel 4 | Beispiel 5 |
|---|---|---|---|---|
| pH-Wert bei Umsetzung | 5,0 ± 0,1 | 6,0 ± 0,1 | 8,0 ± 0,1 | 9,0 ± 0,1 |
| 1,6-Dichlorhexanrestgehalt (ppm) | <10 | <10 | <10 | ca. 11 |
| Umsatz an 1,6-Dichlorhexan (%) | >99,9 | >99,9 | >99,9 | >99,9 |

In allen Beispielen (Beispiele 2 bis 5) war die erhaltenen Reaktionsmischung klar und homogen.

Zur Reinausbeutebestimmung hinsichtlich der Beispiele 2 und 5 wurden diese nochmals wiederholt. Eine GC-Analysenprobe wurde dem jeweiligen Reaktionsgemisch jedoch nicht entnommen. Die Reinausbeute an 1,6-Hexamethylen-bis(Natriumthiosulfat)-Dihydrat betrug für die Wiederholung von Beispiel 2 ca. 91,4%²⁾ und für die Wiederholung von Beispiel 5 ca. 90,6%²⁾.
²⁾ Die Reinausbeute für die Synthese nach den Beispielen 2 und 5 wurde wieder wie in Beispiel 1 durch Umsetzung der jeweils erhaltenen Reaktionslösungen mit wässriger NaBEC-Lösung durchgeführt. Nach dieser Vorgehensweise betrug die Ausbeute an 1,6-Bis(N,N-dibenzylthiocarbamoyldithio)hexan für die Umsetzung nach Beispiel 2 ca. 89,6% der Theorie mit einem Gehalt von ca. 95% und für diejenige nach Beispiel 5 ca. 89,7% der Theorie mit einem Gehalt von ca. 94%. Damit muss die Reinausbeute an 1,6-Hexamethylen-bis(Natriumthiosulfat)-Dihydrat bei der erfindungsgemäßen Umsetzung von 1,6-Dichlorhexan mit Natriumthiosulfatpentahydrat für die Reaktion nach Beispiel 2 ca. 91,4% (89,6% x 0,95 x 1/0,931 = 91,4%) und für diejenige nach Beispiel 5 ca. 90,6% (89,7% x 0,94 x 1/0,931 = 90,6%) betragen haben.

### Beispiel 6 (Vergleichsbeispiel analog EP-A 432 417, Seite 4, Zeilen 50 bis 54)

Man arbeitete wie in Beispiel 1. Es wurde jedoch weder vor noch während der Umsetzung von 1,6-Dichlorhexan eine pH-Wert-Einstellung oder eine pH-Wert-Korrektur durchgeführt. Der pH-Wert wanderte mit Beginn der Umsetzung bei Siedehitze aus dem Bereich von ca. 6 bis 7 nach ca. 2,7 am Ende der Reaktionszeit. Für die GC-Analyse wurde eine Probe aus dem gelblichen, leicht opak aussehenden Reaktionsgemisch entnommen. An dieser Probe wurde ein Restgehalt an 1,6-Dichlorhexan von ca. 0,9% (9000 ppm) bestimmt, was einem Umsatz von nur ca. 89% entspricht.

Zur Bestimmung der Ausbeute wurde der Versuch ohne Probennahme für eine GC-Untersuchung wiederholt. Der pH-Wert lag am Ende dieses Wiederholungsversuches in der Siedehitze bei ca. 1,8. Unumgesetztes 1,6-Dichlorhexan wurde azeotrop bei Normaldruck zusammen mit Wasser aus dem Reaktionsgemisch abdestilliert. Die abdestillierte Gesamtmasse an Dihalogenid und Wasser von ca. 120 g wurde in Form von vollentsalztem Wasser dem Ansatz wieder zugefügt. Die Reinausbeute an 1,6-Hexamethylen-bis(Natriumthiosulfat)-Dihydrat lag ca. 16,4%³⁾.
³⁾ Die Reinausbeutebestimmung wurde wieder wie in Beispiel 1 durch Umsetzung der erhaltenen Reaktionslösung mit wässriger NaBEC-Lösung durchgeführt. Es wurden ca. 44,8 g (25,8% der Theorie) klebriger Feststoff mit einem Gehalt an 1,6-Bis(N,N-dibenzyl-thiocarbamoyldithio)hexan von ca. 52% erhalten. Ölig gelbes Material, das sich beim Abnutschen in der Mutterlauge abschied, wurde durch Extraktion mit Toluol abgetrennt. Nach dem Abdampfen des Toluols wurden ca. 19,1 g (11,0% der Theorie) zähes, gelbes Öl erhalten, das laut HPLC einen Gehalt von ca. 17% an 1,6-Bis(N,N-dibenzylthiocarbamoyl-dithio)hexan aufwies. Daraus ergibt sich eine Gesamt-Reinausbeute an 1,6-Bis(N,N-dibenzylthiocarbamoyldithio)hexan von ca. 15,3%. Damit muss die Reinausbeute an 1,6-Hexamethylen-bis(Natriumthiosulfat)-Dihydrat bei der Umsetzung von 1,6-Dichlorhexan mit Natriumthiosulfatpentahydrat nach Stand der Technik ca. 16,4% (15,3% x 1/0,931 = 16,4%) betragen haben.

Bewertung: Nach diesem Stand der Technik wird 1,6-Hexamethylen-bis(Natriumthiosulfat)-Dihydrat in völlig unzureichender Reinausbeute erhalten.

### Beispiel 7 (Vergleichsbeispiel bei pH 10)

Man arbeitete wie in Beispiel 1, führte die Umsetzung von 1,6-Dichlorhexan von Anfang bis Ende jedoch bei einem pH-Wert von 10,0 ± 0,1 durch. Die schwach saure Mischung wurde vor dem Start der Reaktion mit einigen Tropfen 2,5%iger Natronlauge auf pH 10 eingestellt. Nach beendeter Reaktionszeit waren ca. 135 ml an 2,5%iger Natronlauge verbraucht. Die fertige Reaktionsmischung zeigte in der Siedehitze etwas weißen Niederschlag. Die GC-Analyse am fertigen Reaktionsgemisch ergab einen Restgehalt an 1,6-Dichlorhexan von ca. 23 ppm, was einem Umsatz von >99,9% entspricht.

Die Reaktion wurde nochmals wiederholt, jedoch ohne eine GC-Analysenprobe zu entnehmen. Die Reinausbeute an 1,6-Hexamethylen-bis(Natriumthiosulfat)-Dihydrat lag bei ca. 63,1%⁴⁾..
⁴⁾ Die Reinausbeutebestimmung wurde wieder wie in Beispiel 1 durch Umsetzung der erhaltenen Reaktionslösung mit wässriger NaBEC-Lösung durchgeführt. Es wurde ein sehr feiner Niederschlag erhalten, dessen Isolierung aus dem Reaktionsgemisch mittels Nutsche mehrere Stunden beanspruchte. Vor diesem Hintergrund wurde der Feststoff auf der Nutsche nicht gewaschen. Statt dessen wurde der Nutschkuchen ausgetragen, mit gesättigter Natriumchloridlösung aufgeschlämmt und dann mit Toluol extrahiert. Die wässrige Phase wurde nochmals mit Toluol extrahiert. Die vereinigten toluolischen Phasen wurden zunächst mit gesättigter Natriumchloridlösung und dann mit vollentsalztem Wasser gewaschen. Nach dem Verdampfen des Toluols am Rotationsverdampfer im Vakuum und anschließend im Vakuumtrockenschrank bei 50°C bis zur Gewichtskonstanz getrocknet wurden ca. 114,3 g (66,0% der Theorie) zähes Öl erhalten, das nach längerer Zeit kristallisierte. Der Gehalt an 1,6-Bis(N,N-dibenzylthiocarbamoyldithio)hexan betrug ca. 89%. Damit muss die Reinausbeute an 1,6-Hexamethylen-bis(Natriumthiosulfat)-Dihydrat bei der Umsetzung von 1,6-Dichlorhexan mit Natriumthiosulfatpentahydrat ca. 63,1% (66,0% x 0,89 x 1/0,931 = 63,1%) betragen haben.

Bewertung: Oberhalb des erfindungsgemäßen pH-Wert-Bereichs wird 1,6-Hexamethylenbis(Natriumthiosulfat)-Dihydrat in nur unzureichender Reinausbeute erhalten.

## Patentansprüche

1. Verfahren zur Herstellung der Verbindungen der Formel (I)
Me¹ O₃S S-(CH₂)ₙ-S SO₃ Me² (I),
worin Me¹ und Me² gleich oder verschieden sind und einwertige Metallionen oder Ammoniumionen bedeuten und n eine ganze Zahl von 2 bis 8 darstellt,
**dadurch gekennzeichnet, dass** man Verbindungen der Formel (II)
X-(CH₂)ₙ-X (II),
worin X Halogen bedeutet und n die gleiche Bedeutung wie in Formel (I) hat,
mit Thiosulfationen bei einer Reaktionstemperatur von 80° bis 150°C umsetzt, wobei während der Umsetzung in Wasser ohne Zusatz von Alkoholen und/oder Glykolen ein pH-Wert-Bereich von 3 bis 9,8 eingestellt wird.

2. Verfahren nach Anspruch 1, **dadurch gekennzeichnet, dass** man während der Umsetzung einem pH-Bereich von 4 bis 9,4 einstellt.

3. Verfahren nach Ansprüchen 1 und 2, **dadurch gekennzeichnet, dass** man die Umsetzung bei Temperaturen von 85°C bis 140°C durchführt.

4. Verfahren nach Ansprüchen 1 bis 3, **dadurch gekennzeichnet, dass** man 200 bis 250 Mol-% Thiosulfat, bezogen auf Mole an eingesetzten organischen Dihalogenverbindungen der Formel (II), einsetzt.

## Claims

1. Process for preparing the compounds of the formula (I)
Me¹ O₃S S-(CH₂)ₙ-S SO₃ Me² (I)
where Me¹ and Me² are the same or different and are each monovalent metal ions or ammonium ions and n is an integer from 2 to 8,
**characterized in that** compounds of the formula (II)
X-(CH₂)ₙ-X (II)
where X is halogen and n is as defined in formula (I) are reacted with thiosulphate ions at a reaction temperature of 80° to 150°C, a pH range of 3 to 9.8 being set during the reaction in water without addition of alcohols and/or glycols.

2. Process according to Claim 1, **characterized in that** a pH range of 4 to 9.4 is set during the reaction.

3. Process according to Claims 1 and 2, **characterized in that** the reaction is carried out at temperatures of 85°C to 140°C.

4. Process according to Claims 1 to 3, **characterized in that** 200 to 250 mol% of thiosulphate is used based on moles of organic dihalogen compounds of the formula (II) used.

## Revendications

1. Procédé de fabrication des composés de formule (I)
Me¹ O₃S S-(CH₂)ₙ-S SO₃ Me² (I)
dans laquelle Me¹ et Me² sont identiques ou différents et signifient des ions métalliques monovalents ou des ions ammonium, et n représente un nombre entier de 2 à 8,
**caractérisé en ce que** des composés de formule (II)
X-(CH₂)ₙ-X (II)
dans laquelle X signifie un halogène et n a la même signification que dans la formule (I),
sont mis en réaction avec des ions thiosulfate à une température de réaction de 80 à 150 °C, un pH dans la plage allant de 3 à 9,8 étant ajusté pendant la réaction dans de l'eau sans ajout d'alcools et/ou de glycols.

2. Procédé selon la revendication 1, **caractérisé en ce qu'**un pH dans la plage allant de 4 à 9,4 est ajusté pendant la réaction.

3. Procédé selon les revendications 1 et 2, **caractérisé en ce que** la réaction est réalisée à des températures de 85 °C à 140 °C.

4. Procédé selon les revendications 1 à 3, **caractérisé en ce que** 200 à 250 % en moles de thiosulfate, par rapport aux moles de composés dihalogénés organiques de formule (II) utilisés, sont utilisés.
